# EUROPÄISCHE PATENTSCHRIFT

(11) **EP 2 934 397 B1**
(45) Veröffentlichungstag und Bekanntmachung des Hinweises auf die Patenterteilung: **30.05.2018**
(21) Anmeldenummer: 13820762.6
(22) Anmeldetag: 19.12.2013
(51) Int. Cl.: A61F 2/50, A61F 2/78, A61F 5/01, A61B 5/00, A61B 5/107, A61B 34/10

(54) **LEIT-STÜTZ-STRUKTUR ZUM KOPPELN MIT EINEM LEBEWESEN UND VERFAHREN ZUR BESTIMMUNG GEEIGNETER ANLAGEFLÄCHEN AN DEM LEBEWESEN**
GUIDING/SUPPORTING STRUCTURE FOR COUPLING TO A LIVING BEING, AND METHOD FOR DETERMINING SUITABLE CONTACT SURFACES ON THE LIVING BEING
STRUCTURE DE GUIDAGE ET DE SUPPORT À APPLIQUER SUR UN ÊTRE VIVANT ET PROCÉDÉ PERMETTANT DE DÉTERMINER DES SURFACES D'APPUI APPROPRIÉES SUR L'ÊTRE VIVANT

(30) Priorität: 21.12.2012 DE 102012025431
(43) Veröffentlichungstag der Anmeldung: 28.10.2015
(73) Patentinhaber: Exos GmbH, 80797 München (DE)
(72) Erfinder: DIEZ, Laura, 87439 Kempten (DE); JUNIOR, Volker, 82166 Gräfelfing (DE); STEHLING, Greta, 83101 Thansau (DE)
(74) Vertreter: Ascherl, Andreas
(86) Internationale Anmeldenummer: PCT/EP2013/077460
(87) Internationale Veröffentlichungsnummer: WO 2014/096235

(56) Entgegenhaltungen:
- EP-A1- 1 843 291
- WO-A1-2008/092617
- DE-A1-102008 032 992
- Dipl. Ing. Michael Hasenpusch: "Beitrag zur Optimierung der Stumpfdatenerfassung und computergestützten Stumpfbettgestaltung von Unterschenkelprothesen", 1. Januar 1997 (1997-01-01), Technische Universität Berlin, Berlin, XP7922626, Seiten 116-142, das ganze Dokument
- ANA RITA DOMINGUES ET AL: "SKIN STRAIN FIELD ANALYSIS OF THE HUMAN ANKLE JOINT", 4O CONGRESSO NACIONAL DE BIOMECÀNICA, 5 February 2010 (2010-02-05), XP055288712, Coimbra, Portugal

## Beschreibung

Die vorliegende Erfindung bezieht sich auf ein Verfahren zum Erfassen von Oberflächenverformungen von Körperflächen von Lebewesen zur Bestimmung geeigneter Anlageflächen für mit der Oberfläche der Lebewesen in Kontakt bringbare Leit-Stütz-Strukturen, insbesondere Orthesen, sowie auf eine Leit-Stütz-Struktur zur Kopplung mit einem Lebewesen.

Die Kopplung von Leit-Stütz-Strukturen mit Lebewesen, insbesondere mit Menschen, erfordert die Berücksichtigung vielfältiger Überlegungen. Gegensätzlich zur Anbringung von Leit-Stütz-Strukturen an z.B. mechanischen Vorrichtungen oder an Gebäuden sind Körper von Lebewesen stets voneinander verschieden. Dies resultiert z.B. aus unterschiedlichen Körpergrößen, aus unterschiedlichen Muskel- und Gewebestrukturen, aus unterschiedlichen Längenverhältnissen der Beine zu dem Oberkörper und aus vielem mehr. Gegensätzlich zu einer Anbindung einer Leit-Stütz-Struktur an einer mechanischen Vorrichtung resultiert bereits daraus in leicht erkennbarer Weise eine enorme Komplexität.

Ferner ist zu berücksichtigen, dass Lebewesen Schmerzen empfinden und die Kontaktstellen, an denen eine Leit-Stütz-Struktur angebracht bzw. anbringbar ist, wund werden können. Somit sind die Leit-Stütz-Strukturen auch derart zu gestalten, dass sie keine Einschnürungen von Körperregionen bewirken, die zu Schmerzen führen würden. Dennoch soll die Leit-Stütz-Struktur an der angekoppelten Körperregion nicht verrutschen, was häufig als sehr störend beurteilt wird und wodurch die Funktionsfähigkeit einer Leit-Stütz-Struktur zumeist eingeschränkt oder gemindert wird. Ferner soll eine Leit-Stütz-Struktur möglichst keine unbeabsichtigte Behinderung des Bewegungsablaufs der Person bewirken.

Die Druckschrift DE10200S008605A1 offenbart ein Verfahren zur Herstellung einer äußeren Prothese oder Orthese. Es erfolgt dabei die Erstellung einer Schichtaufnahme eines Körperteils, die Umwandung der erstellten Daten in ein 3D-Modell, die Auswertung der Knochen-, Muskel, und Fettgewebestruktur, die Ermittlung von Kompressionszonen in Abhängigkeit von der Auswertung und die Erstellung eines Prothesen-/Orthesenelements in Abhängigkeit von diesen Daten. In der DE102005008605A1 wird dabei die Erkenntnis, dass im Falle von Amputationen einzelne Muskeln ihren Ansatz verlieren und sich über die Zeit die Muskelmasse abbaut und durch Fettmasse ersetzt wird, für die Gestaltung von Prothesen berücksichtigt. Es erfolgt somit gemäß der DE102005008605A1 eine Analyse der inneren Struktur eines Körperteils. Die Anbindung der Prothese an den Körper erfolgt über eine möglichst großflächige Anbindung. Die zur Anbindung verwendeten Flächenelemente sind dabei nach Regelgeometrien gestaltet.

Auch die Druckschrift EP 1 843 291 A1 offenbart ein Verfahren, gemäß dem anhand einer Vielzahl an Querschnittsaufnahmen eines Extremitäten-Stumpfes eine Separierung der Anteile von Knochen-, Muskel- und Fettgewebe erfolgt. Hierzu ähnliche Verfahren werden durch die Druckschriften WO2008/092617A1 und DE102008032992A1 offenbart. Ein Verfahren zur Bestimmung von ortsfesten Punkten auf einer Oberfläche wird in dem Beitrag "Skin Strain Field Analysis of the Human Ankle Joint" von Ana Rita Domingues et al, im Rahmen des 4° Congresso Nacional de Biomecänica, Coimbra (Portugal), vom 5. Februar 2010, offenbart. Ferner offenbart Dipl.-Ing. Michael Hasenpusch in "Beitrag zur Optimierung der Stumpfdatenerfassung und computergestützten Stumpfbettgestaltung in Unterschenkelprothesen", 1. Januar 1997 (1997-01-01), Technische Universität Berlin, Berlin, XP7922626, Seiten 116-142 ein Verfahren, gemäß dem für verschiedene Belastungszustände lokale Flächenpressungen zwischen einem Körperteil und einer Prothese ermittelt werden.

Die aus dem Stand der Technik bekannten Verfahren weisen den Nachteil auf, dass sie im Wesentlichen nur für die Herstellung von Prothesen bzw. Orthesen geeignet sind, die mit Körperteilen gekoppelt werden, in denen keine oder nur noch eine geringe Muskelaktivität erkennbar ist. Die Übertragung dieser Verfahren bzw. daraus erzeugter Orthesen auf Körperteile mit normaler Muskelaktivität würde bei einer Anspannung der Muskulatur und einer sich daraus ergebenden lokalen Volumenvergrößerung des Muskels zu einem extrem hohen Anpressdruck innerhalb der umschließenden Orthese führen. Aus diesem Druckanstieg würden sich Einschnürungen des Körperteils und daraus resultierende Schmerzen bzw. Leistungseinbußen bzw. unerwünschte Bewegungseinschränkungen für die Person ergeben, die eine solche Orthese trägt.

Es ist daher die Aufgabe der vorliegenden Erfindung ein Verfahren und eine entsprechende Leit-Stütz-Struktur bereitzustellen, die mit einem Körperteil eines Lebewesens koppelbar ist und bei einer normalen Muskelaktivität in dem Körperteil eine bestmögliche Funktionalität, insbesondere einen hohen Tragekomfort, insbesondere mit deutlich weniger Einschnürungen als bei den aus dem Stand der Technik bekannten Prothesen und Orthesen, ermöglicht.

Die zuvor genannte Aufgabe wird gemäß dem Gegenstand von Anspruch 1 durch ein Verfahren zum Erfassen von Oberflächenverformungen von Körperflächen von Lebewesen zur Bestimmung geeigneter Anlageflächen für mit der Oberfläche der Lebewesen in Kontakt bringbare Leit-Stütz-Strukturen gelöst. Als Lebewesen werden hierbei bevorzugt Menschen, insbesondere Kinder, Erwachsene, Senioren und/oder Körperbehinderte und/oder Verletzte und/oder Sportler, und/oder Tiere, insbesondere Haustiere, wie Hunde und/oder Katzen, oder Nutztiere, wie Pferde, Kühe und/oder Schweine, verstanden.

Ein solches Verfahren umfasst mindestens die Schritte des Ausrichtens zumindest eines Körperteils des Lebewesens gegenüber einer Erfassungseinrichtung zum zumindest teilweisen Erfassen der Oberflächenverformungen von zumindest einem Oberflächenbereich des Körperteils, des Erfassens einer ersten Oberflächencharakteristik bzw. -form des Oberflächenbereichs mittels der Erfassungseinrichtung, wobei sich das Körperteil in einem ersten Belastungszustand, insbesondere einem Zustand entspannter Muskulatur und/oder in einem Zustand zueinander in einer Neutralstellung ausgerichteter Körperteile, befindet, des Erfassens einer zweiten Oberflächencharakteristik bzw. -form des Oberflächenbereichs mittels der Erfassungseinrichtung, wobei sich das Körperteil in einem vom ersten Belastungszustand verschiedenen zweiten Belastungszustand, insbesondere einem Zustand angespannter Muskulatur und/oder in einem Zustand zueinander geneigter und/oder rotierter und/oder geschwenkter Körperteile, befindet, und des Bestimmens der sich zwischen dem ersten Belastungszustand und dem zweiten Belastungszustand ergebenden lokalen Oberflächenverformungen, insbesondere mittels einer Auswertung der erfassten Oberflächencharakteristika bzw. -flächen bzw. durch einen Abgleich der erfassten Oberflächenformen, insbesondere durch einen Abgleich der ersten Oberflächenform mit der zweiten Oberflächenform.

Eine Leit-Stütz-Struktur kann bevorzugt neben anderem konkret als eine Orthese, insbesondere Arm-, Knie-, Gelenk-, Bein-, Hüft-, Schulter-, Rumpf-, Hals-, Nacken-, Hand-, Fußorthese, ein Prothese, ein Exoskelet, ein Trägersystem, insbesondere eines Rucksacks, eine Tasche, ein Klettergurt, ein Hebesystem, ein Rucksack, eine Gehhilfe, insbesondere eine Krücke, ein Schuh, insbesondere ein Skischuh, eine Schutzeinrichtung, wie z.B. eine Bandage, insbesondere für Sporteinsätze, oder ein Protektor, insbesondere zur Vermeidung von Arbeitsverletzungen und/oder Sportverletzungen, eine Stabilisierungseinrichtung, wie eine Schiene zum Schienen von Knochenbrüchen, etc. und/oder als eine Kombinationen daraus verstanden werden.

Als Erfassungseinrichtung kann bevorzugt eine oder können besonders bevorzugt mehrere identische oder voneinander verschiedene Einrichtungen bezeichnet sein. Besonders bevorzugt erfolgt die Erfassung der Oberflächenverformungen bzw. variierender Körperteilvolumina mittels mit dem Körperteil bzw. den Oberflächenbereichen koppelbaren Sensoreinrichtungen, die insbesondere zur Druckerfassung ausgebildet sind, und/oder einer optischen Erfassungseinrichtung, insbesondere einer Bilderfassungseinrichtung oder einer Videoerfassungseinrichtung, und/oder mittels einer Emittiereinrichtung zum Ausgeben von Strahlen, die das Körperteil durchdringenden, und/oder einer mechanischen und/oder fluidischen Erfassungseinrichtung und/oder ein Terahertz-Scanner. Wobei die Sensoreinrichtungen bevorzugt flächig um das Körperteil herum anbringbar sind, insbesondere an einem hosenartigen Mittel angeordnet sind, und wobei die Bilderfassungseinrichtung bevorzugt ein 3D-Scanner ist und wobei die Emittiereinrichtung bevorzugt ein Röntgengerät, ein Computertomograph oder ein Magnetresonanztomograph ist.

Das Ausrichten eines Körperteils gegenüber einer Erfassungseinrichtung ist bevorzugt als Einbringen des Körperteils in den Wirkbereich der Erfassungseinrichtung und/oder als Überführen des Körperteils in eine konkrete Position gegenüber der Erfassungseinrichtung zu verstehen.

Ein zumindest abschnittsweises Erfassen von Oberflächenverformungen beschreibt bevorzugt, dass nicht alle Oberflächenverformungen eines Körperteils erfasst werden müssen, wobei besonders bevorzugt alle Oberflächenverformungen eines Körperteils erfasst werden.

Die Bezeichnung Oberflächencharakteristik beschreibt hierbei bevorzugt die Oberflächenstruktur bzw. die Topographie der Oberfläche, insbesondere der Haut und/oder des Fells des Lebewesens. Besonders bevorzugt ist dies makroskopisch zu verstehen, d.h. die sich aus Verformungen, insbesondere Ausdehnungen und Kontraktionen, von z.B. mehr als 1mm und bevorzugt von mehr als 3mm und besonders bevorzugt von mehr als 5mm ergebende Charakteristik bzw. Struktur bzw. Form erfasst wird.

Dieses Verfahren ist vorteilhaft, da durch die Bestimmung der lokalen Oberflächenverformungen Oberflächenbereiche identifizierbar sind, die trotz normaler Muskelaktivität und sogar bei sportlichen Aktivitäten im Vergleich zu anderen Oberflächenbereichen desselben Körperteils deutlich geringere oder keine Verformungen erfahren. An diesen gering verformten oder unverformten Oberflächenbereichen sind Leit-Stütz-Strukturen in vorteilhafter Weise ankoppelbar, da die Gefahr von Einschnürungen, aufgrund der deutlich geringeren Ausdehnungen, stark reduziert ist. Die Leit-Stütz-Strukturen können daher mit deutlich geringeren Kräften an dem jeweiligen Körperteil angekoppelt werden. Weiterhin wird durch die Anbringung einer Leit-Stütz-Struktur an den gering verformten bzw. unverformten Oberflächenbereichen ein Verrutschen der Leit-Stütz-Struktur nahezu ausgeschlossen, wodurch in vorteilhafter Weise eine Erhaltung der Bewegungsfreiheit gewährleistet und/oder die intendierte bzw. definierte bzw. vorgesehene Bewegungseinschränkung gewährleistet wird.

Bevorzugt dient das Verfahren somit zur Bestimmung von Oberflächenbereichen eines Körperteils eines Lebewesens, die zur Inkontaktbringung mit Leit-Stütz-Strukturen zur unidirektionalen oder bidirektionalen Übertragung von Kräften und/oder Momenten geeignet sind, wobei sich die Oberflächenbereiche bevorzugt dadurch auszeichnen, dass sie im Vergleich zu anderen Oberflächenbereichen desselben Körperteils geringere Verformungen zwischen zwei Belastungszuständen aufweisen bzw. zur Bestimmung von sich zwischen verschiedenen Belastungszuständen statisch bzw. im Wesentlichen statisch verhaltenden Oberflächenanteilen bzw. zur Bestimmung, an welcher Position des erfassten Oberflächenbereichs gegenüber einem Ausgangszustand, welcher Grad an Verformung auftritt.

Das Verfahren kann bevorzugt zur Unterstützung der Herstellung einer Leit-Stütz-Struktur und besonders bevorzugt für ein Herstellungsverfahren zur Herstellung von Leit-Stütz-Strukturen übernommen oder angepasst werden.

Gemäß einer bevorzugten Ausführungsform der vorliegenden Erfindung wird ein Abgleich der erfassten lokalen Oberflächenverformungen mit zumindest einem definierten Schwellenwert oder einer definierten Schwellenwertfunktion durchgeführt, wobei daraus resultierende Verformungswerte einerseits, insbesondere unterhalb des definierten Schwellenwerts oder der definierten Schwellenwertfunktion, als Werte geringer Oberflächenverformung, insbesondere geringer Expansion oder Kontraktion, beschreibbar, definierbar und/oder festlegbar sind und andererseits, insbesondere oberhalb des definierten Schwellenwerts oder der definierten Schwellenwertfunktion, als Werte hoher Oberflächenverformung beschreibbar, definierbar und/oder festlegbar sind.

Es ist hierbei denkbar, dass nicht nur ein Schwellenwert und/oder eine Schwellenwertfunktion zum Identifizieren von Bereichen geringer und hoher Verformung vorgesehen sind, sondern mehrstufig, insbesondere drei-, vier-, fünfstufig, insbesondere mehr als fünfstufig, verschiedene Grade an Dynamik bzw. Statik identifizierbar sind. So können z.B. alle Bereiche deren Verformung sich unterhalb eines ersten Schwellenwertes befinden als statisch bezeichnet werden, alle Bereiche deren Verformungen oberhalb des ersten Schwellenwertes jedoch unterhalb eines zweiten Schwellenwertes liegen als teilweise dynamisch bezeichnet werden und alle Bereiche deren Verformungen oberhalb des zweiten Schwellenwertes liegen als dynamisch bezeichnet werden. Die unterschiedlichen Bereiche können z.B. zur optischen Ausgabe speziell gekennzeichnet sein, insbesondere können sie entsprechend einem zu dem jeweiligen Grad an Verformung zugeordneten Farbschema eingefärbt sein.

Diese Ausführungsform ist vorteilhaft, da durch den Schwellenwert bzw. die Schwellenwerte und/oder die Schwellenwertfunktion bzw. die Schwellenwertfunktionen die ermittelten Ergebnisse z.B. einer das Verfahren ausführenden bzw. begleitenden Person und/oder für Dokumentationszwecke optisch aufbereitet und somit leicht verständlich ausgebbar sind. Weiterhin ist denkbar, dass automatisiert anhand der mittels der Schwellenwerte und/oder Schwellenwertfunktionen bestimmten optimalen Kopplungsbereiche von einer Prozessoreinrichtung eine Ansicht, insbesondere eine 3D-Ansicht des Körperteils und/oder der Leit-Stütz-Struktur, und/oder ein Fertigungsplan generiert wird und/oder die Fertigung einer Leit-Stütz-Struktur angestoßen bzw. umgesetzt oder direkt abgeleitet wird.

Die Bestimmung der lokalen Oberflächenverformungen und/oder der Abgleich der erfassten Oberflächenverformungen wird gemäß einer weiteren bevorzugten Ausführungsform durch eine Prozessoreinrichtung bewirkt. Die Prozessoreinrichtung kann dabei z.B. Bestandteil der Erfassungseinrichtung sein und/oder mit der Erfassungseinrichtung gekoppelt sein bzw. koppelbar sein.

Bevorzugt erfolgt die Bestimmung und/oder der Abgleich mittels der Prozessoreinrichtung vollautomatisch oder teilautomatisch.

Die Prozessoreinrichtung umfasst bevorzugt eine Speichereinrichtung zum Abspeichern von Rohdaten und/oder aufbereiteten Daten, wobei auch denkbar ist, dass die Speichereinrichtung und die Prozessoreinrichtung lediglich signaltechnisch, d.h. für den Austausch von Daten, miteinander verbunden sind. Zusätzlich oder alternativ ist die Prozessoreinrichtung bevorzugt mit einer Ausgabeeinrichtung, wie einem Bildschirm, einem Drucker, einem optischen Laufwerk, einer direkt ansteuerbaren Fertigungseinrichtung, insbesondere einem 3D-Drucker, etc. verbunden. Ferner ist die Prozessoreinrichtung bevorzugt mit einem Aufbereitungsmittel ausgestattet bzw. hat Zugriff auf ein solches Aufbereitungsmittel, insbesondere ein Computer-aided Design-Tool, mittels dem die Verformungswerte in zur Konstruktion bzw. zur Gestaltung einer Leit-Stütz-Struktur geeignete Daten- bzw. Dateiformate überführbar sind.

Gemäß einer weiteren bevorzugten Ausführungsform werden in Abhängigkeit von mindestens zwei definierten Schwellenwerten oder Schwellenwertfunktionen mindestens drei Oberflächenbereiche Zonen unterschiedlicher Oberflächenverformungen zugeordnet, insbesondere sind die Oberflächenverformungen in der dem ersten Oberflächenbereich zuordenbaren ersten Zone geringer als die Oberflächenverformungen in der dem zweiten Oberflächenbereich zuordenbaren zweiten Zone und geringer als die Oberflächenverformungen in der dem dritten Oberflächenbereich zuordenbaren dritten Zone, wobei die Oberflächenverformungen in der dritten Zone in Bezug zu einem Zentrum des Körperteils bevorzugt entgegengerichtet zu den Oberflächenverformungen in der zweiten Zone erfolgen. Es ist jedoch ebenfalls denkbar, dass die Oberflächenverformungen in der dem ersten Oberflächenbereich zuordenbaren ersten Zone größer als die Oberflächenverformungen in der dem zweiten Oberflächenbereich zuordenbaren zweiten Zone und geringer als die Oberflächenverformungen in der dem dritten Oberflächenbereich zuordenbaren dritten Zone sind.

Gemäß einer weiteren bevorzugten Ausführungsform der vorliegenden Erfindung wird eine zu den Verformungswerten und/oder zu den einerseits des Schwellenwerts oder der Schwellenwertfunktion zuordenbaren Oberflächenbereichen korrespondierende Darstellung zum Anzeigen unterschiedlicher Verformungsgrade an unterschiedlichen Positionen des Oberflächenbereichs erzeugt und/oder visualisiert, wobei besonders bevorzugt die Verformungswerte und/oder die einerseits des Schwellenwerts oder der Schwellenwertfunktion zuordenbaren Oberflächenbereiche in Daten zur Erstellung eines 3D-Modells, insbesondere eines Körperteils und/oder einer an das Körperteil angepassten Leit-Stütz-Struktur, umgewandelt bzw. überführt werden.

Diese Ausführungsform ist vorteilhaft, da die ermittelten Ergebnisse einer das Verfahren ausführenden bzw. begleitenden Person und/oder für Dokumentationszwecke optisch aufbereitet und somit leicht verständlich ausgebbar sind. Ferner kann anhand der optischen Aufbereitung unmittelbar eine Verifizierung der ausgegebenen Verformungswerte, insbesondere durch eine die Erfassungseinrichtung bedienende Person, erfolgen.

Ferner kann sich die vorliegende Erfindung auf ein Computerprogrammprodukt beziehen, das einen Programmcode umfasst, der auf einem computerlesbaren Medium gespeichert ist, um zumindest einzelne der zur Erfassung der Verformungswerte und/oder deren Verarbeitung erforderlichen und zuvor bzw. im Folgenden beschriebenen Verfahrensschritte auszuführen, wenn das Computerprogrammprodukt auf einem Computer ausgeführt wird bzw. bezieht sich auf ein computerimplementiertes Verfahren nach einem der Ansprüche 1 bis 5. Es ist jedoch ebenfalls denkbar, dass sich die vorliegende Erfindung auf ein Steuerungsmittel bzw. eine Steuerungssoftware, insbesondere zur Ansteuerung der Erfassungseinrichtung und/oder der Prozessoreinrichtung und/oder einer Fertigungseinrichtung, bezieht.

Weiterhin wird die zuvor genannte Aufgabe der Erfindung ebenfalls durch eine Leit-Stütz-Struktur, insbesondere eine Orthese, zur Kopplung mit einem Lebewesen und zum Übertragen von in einen ersten Anteil der Leit-Stütz-Struktur einleitbaren Kräften auf einen zweiten vom ersten Anteil beabstandeten Anteil der Leit-Stütz-Struktur gelöst. Der erste Anteil der Leit-Stütz-Struktur ist zum zumindest mittelbaren und zumindest abschnittsweise flächigen Anliegen an der Oberfläche eines Körperteils eines Lebewesens ausgebildet und die Oberfläche des Körperteils ist zumindest abschnittsweise von dem flächigen Abschnitt kontaktierend überlagerbar. Ferner ist zumindest der flächige Abschnitt des ersten Anteils zum Anliegen an dem Körperteil derart ausgebildet, dass er in einer für die Funktionserfüllung der Leit-Stütz-Struktur vorgesehenen definierten Ausrichtung gegenüber dem Körperteil an Oberflächenbereiche anlegbar ist, die beim Übergang von einem ersten Belastungszustand in einen zweiten Belastungszustand Oberflächenverformungen aufweisen, die während desselben Übergangs geringer sind als Oberflächenverformungen weiterer Oberflächenbereiche des Körperteils oder er in einer für die Funktionserfüllung der Leit-Stütz-Struktur vorgesehenen definierten Ausrichtung gegenüber dem Körperteil an Oberflächenbereiche anlegbar ist, die beim Übergang von einem ersten Belastungszustand in einen zweiten Belastungszustand Oberflächenverformungen aufweisen, die während desselben Übergangs höher sind als Oberflächenverformungen weiterer Oberflächenbereiche des Körperteils.

Der flächige Abschnitt weist bevorzugt mehrere, insbesondere genau zwei, zwei oder mehr als zwei, insbesondere genau drei, drei oder mehr als drei, insbesondere genau vier, vier oder mehr als vier, Bereiche auf, mittels denen die Leit-Stütz-Struktur besonders bevorzugt formschlüssig mit dem Körperteil bzw. den Körperteilen koppelbar ist.

Weiterhin ist der erste Anteil bevorzugt nicht nur zum mittelbaren und abschnittsweise flächigen Anliegen an der Oberfläche eines Körperteils ausgebildet, sondern kann auch unmittelbar am Körper anliegen, wobei auch denkbar ist, dass der erste Anteil nicht nur abschnittsweise flächig an der Oberfläche des Körperteils anliegt, sondern im Wesentlichen vollständig oder vollständig flächig an dem Körperteil anliegt. Der erste Anteil der Leit-Stütz-Struktur weist bevorzugt ein festes Material bzw. eine Kombination aus mehreren Materialien, insbesondere Carbon, PE, PP etc., auf, die bevorzugt der Leit-Stütz-Struktur ihre Festigkeit verleiht. Dieses Material bzw. diese Materialkombination steht aber in dem mit dem Körperteil gekoppelten Zustand bevorzugt nicht mit dem Körperteil bzw. der Oberfläche des Körperteils in Kontakt. Der erste Anteil der Leit-Stütz-Struktur weist bevorzugt ferner ein weiches Material oder eine Kombination aus Materialien, insbesondere Schaumstoff, Textilien, Gummi, etc., auf, die weicher sind als die festen Materialien und bevorzugt mit der Oberfläche des Körperteils zumindest mittelbar und bevorzugt unmittelbar in Kontakt bringbar sind.

Eine solche Leit-Stütz-Struktur, die bevorzugt als Orthese ausgebildet ist, weist gegenüber den aus dem Stand der Technik bekannten Orthesen signifikante Vorteile auf. So ist die erfindungsgemäße Leit-Stütz-Struktur neben anderem insbesondere zur Verletzungsprävention im Sportbereich einsetzbar, da aufgrund der erfindungsgemäßen Anpassung an das entsprechende Körperteil bzw. an die entsprechenden Körperteile im Vergleich zu den aus dem Stand der Technik bekannten Orthesen keinerlei bzw. deutlich geringe bzw. zu vernachlässigende Einschnürungen des Körperteils bzw. der Körperteile, insbesondere im Kopplungsbereich, d.h. zwischen der Leit-Stütz-Struktur und der Körperoberfläche, bei unterschiedlichen Belastungszuständen, insbesondere Muskelanspannungszuständen, auftreten. Die erfindungsgemäße Leit-Stütz-Struktur dient somit bevorzugt zur ergonomischen Aufnahme von Kräften bzw. zur Übertragung von Kräften, Momenten und/oder Bewegungen vom Körper eines Lebewesens, insbesondere von einem menschlichen Körper, auf die am Körper angebrachte Leit-Stütz-Struktur und/oder andersherum. Die erfindungsgemäße Leit-Stütz-Struktur ist daher für die Verletzungsprävention bei erhöhten Belastungen, wie z.B. im Breitensport oder sogar im Leistungssport, oder in der Unfallprävention, insbesondere bei langen, d.h. z.B. mehrstündigen oder dauerhaften, Arbeitseinsätzen oder als Teil eines Exoskelets oder im Bereich der Rehabilitationseinrichtungen, insbesondere zum definierten Ausrichten zweier oder mehrerer Körperteile zueinander bzw. zum Ausrichten zweier oder mehrerer Teile eines Körperteils, wie z.B. beim Schienen eines gebrochenen Knochens, deutlich komfortabler einsetzbar als die bekannten Orthesen.

Die Leit-Stütz-Struktur kann neben anderem z.B. mittels Gurten, insbesondere elastischen bzw. flexiblen Gurten, Schnürungen, insbesondere elastischen bzw. flexiblen Schnürungen, an dem jeweiligen Körperteil bzw. an den Körperteilen und/oder an bzw. auf bzw. in Kleidung fixierbar sein. In dem Falle, in dem die Leit-Stütz-Struktur in die Kleidung integriert ist, ist die Leit-Stütz-Struktur bevorzugt unlösbar oder lösbar mit der Kleidung verbunden, bevorzugt ist die Leit-Stütz-Struktur an die Kleidung anklettbar bzw. angeklettet. Die Kleidung kann z.B. eine enganliegende elastische Kleidung sein.

Die mittelbar bzw. unmittelbar mit der Oberfläche des Körperteils in Kontakt bringbaren flächigen Anteile, insbesondere die weichen Bauteile, der Leit-Stütz-Struktur sind bevorzugt an den weiteren Bauteilen, insbesondere den festen Bauteilen, bevorzugt formschlüssig, stoffschlüssig, reibschlüssig und/oder feldschlüssig, wie z.B. angeklebt, angenäht, angenietet, angeschweißt, angeklemmt, angeschraubt, integriert gefertigt, laminiert und/oder durch Kombinationen daraus, angebracht. Der flächige Anteil bzw. die flächigen Anteile der Leit-Stütz-Struktur zum Anliegen an den Körperteilen können auch als körperangepasste Flächen bezeichnet werden, die bevorzugt zum in Kontakt bringen mit jeglichen Körperregionen, -teilen, -stellen und Gliedmaßen gestaltet und/oder gefertigt werden können.

Der flächige Abschnitt ist gemäß einer weiteren bevorzugten Ausführungsform der vorliegenden Erfindung derart gestaltet, dass er überwiegend oder ausschließlich bzw. im Wesentlichen vollständig mit Oberflächenbereichen des Körperteils in Kontakt bringbar ist, die sich beim Übergang von einem ersten Belastungszustand in einen zweiten Belastungszustand geringer verformen als die weiteren Oberflächenbereiche des Körperteils.

Die Flächen der Leit-Stütz-Struktur umschließen daher in vorteilhafter Weise bevorzugt den Körper bzw. das Körperteil ohne die Bewegungsfreiheit oder Muskelvolumenveränderung bzw. Oberflächenverformung des Körperteils einzuschränken, insbesondere nicht in unbeabsichtigter Weise einzuschränken, und minimieren weiterhin die Relativbewegung zwischen der Leit-Stütz-Struktur und dem Körper bzw. dem Körperteil bzw. den Körperteilen.

Der zweite Anteil der Leit-Stütz-Struktur ist gemäß einer weiteren bevorzugten Ausführungsform derart gestaltet, dass er derart an einem weiteren Körperteil, insbesondere an einem Unterschenkel, anlegbar ist, dass er in der für die Funktionserfüllung der Leit-Stütz-Struktur vorgesehenen definierten Ausrichtung gegenüber dem Körperteil, insbesondere einem Oberschenkel, an Oberflächenbereiche des weiteren Körperteils anlegbar ist, die beim Übergang von einem ersten Belastungszustand in einen zweiten Belastungszustand Oberflächenverformungen aufweisen, die gegenüber Oberflächenverformungen weiterer Oberflächenbereiche des weiteren Körperteils während desselben Übergangs geringer sind oder an Oberflächenbereiche des weiteren Körperteils anlegbar ist, die beim Übergang von einem ersten Belastungszustand in einen zweiten Belastungszustand Oberflächenverformungen aufweisen, die gegenüber Oberflächenverformungen weiterer Oberflächenbereiche des weiteren Körperteils während desselben Übergangs höher sind.

Diese Ausführungsform ist vorteilhaft, da die Leit-Stütz-Struktur, insbesondere als Orthese, komfortabel an mehreren Teilen eines Körperteils und/oder an mehreren Körperteilen eines Lebewesens, insbesondere einer Person, ankoppelbar und/oder einsetzbar ist.

Gemäß einer weiteren bevorzugten Ausführungsform der vorliegenden Erfindung ist die Form des flächigen Abschnitts in Abhängigkeit von einem Verfahren nach einem der Ansprüche 1 bis 6 bestimmbar bzw. erfolgt eine Herstellung der Leit-Stütz-Struktur in Abhängigkeit von einem Verfahren nach einem der Ansprüche 1 bis 6.

Die Bestimmung bzw. Gestaltung der Form des flächigen Abschnitts in Abhängigkeit von dem erfindungsgemäßen Verfahren stellt sicher, dass die für das jeweilige Lebewesen bzw. den jeweiligen Einsatzbereich benötigte bzw. erforderliche Leit-Stütz-Struktur bevorzugt optimal, d.h. ganz individuell, zu dem Lebewesen passt und dadurch die Gefahr von Einschnürungen und/oder eventuellen Fehlbelastungen äußerst vorteilhaft reduziert wird.

Die Form des flächigen Abschnitts ist gemäß einer weiteren bevorzugten Ausführungsform der vorliegenden Erfindung in Abhängigkeit von Personengruppen und/oder Körperteilgruppen zuordenbaren und in einer Datensammlung vorgehaltenen lokalen Oberflächenverformungsdaten, die nach einem der Ansprüche 1 bis 6 bestimmt sind, bestimmbar.

Diese Ausführungsform ist vorteilhaft, da z.B. nach einer aus Reihenmessungen abgeleiteten Topologie bzw. Oberflächenstruktur bzw. Oberflächencharakteristik bzw. Oberflächenform bzw. Oberflächenverformungsverteilung auf sehr zeitsparende Weise und somit verhältnismäßig günstig Leit-Stütz-Strukturen gestaltbar und/oder fertigbar sind, die zwar nicht individuell an jedes Lebewesen angepasst sind, dennoch zumindest für bevorzugt definierte Gruppen, wie z.B. Männer, Skifahrer, Beinlänge zwischen 100cm und 110cm, annähernd optimal passen.

Gemäß einer weiteren bevorzugten Ausführungsform der vorliegenden Erfindung bildet der flächige Abschnitt zwei Teilbereiche ausbildet, von denen der Oberflächenbereich des Körperteils zumindest teilweise umschließbar ist und zwischen denen eine Ausnehmung ausgebildet ist, wobei ein der Ausnehmung zuordenbarer Oberflächenbereich beim Übergang von einem ersten Belastungszustand in einen zweiten Belastungszustand größere Oberflächenverformungen aufweist als die von dem flächigen Abschnitt überlagerbaren Oberflächenbereiche bzw. die Ausnehmung ist so gestaltet, dass sich die Muskulatur eines Lebewesens in einem angespannten Zustand in die Ausnehmung hinein erstreckt bzw. zwischen den flächigen Abschnitten hindurch erstreckt bzw. zwischen den flächigen Abschnitten weiter hindurch erstreckt als dies in einem unbelasteten Zustand der Fall ist, oder ein der Ausnehmung zuordenbarer Oberflächenbereich beim Übergang von einem ersten Belastungszustand in einen zweiten Belastungszustand geringere Oberflächenverformungen aufweist als die von dem flächigen Abschnitt überlagerbaren Oberflächenbereiche.

Diese Ausführungsform ist vorteilhaft, da durch die ein Körperteil zumindest teilweise umschließende Ausgestaltung der Leit-Stütz-Struktur, diese bevorzugt formschlüssig an dem Körperteil ankoppelbar ist. Die flächigen Abschnitte sind daher besonders bevorzugt so gestaltet, dass sie bei einem korrekten Einsatz in einer vorgesehenen Verwendung an Flächen geringster Volumenveränderung des Körperteils bzw. geringster Kontaktformänderungen der Oberfläche des Körperteils während einer bestimmten Bewegung oder Aktivität anliegen. Ferner bildet die Ausnehmung einen Bereich aus, der Oberflächenverformungen des Körperteils zulässt, ohne dass Einschnürungen erfolgen.

Die zwei Teilbereiche des flächigen Abschnitts sind gemäß einer weiteren bevorzugten Ausführungsform mittels einer sich im Bereich der Ausnehmung erstreckenden Komponente, insbesondere einer zumindest teilweise elastischen und/oder flexiblen Komponente, miteinander verbunden. Bevorzugt ist die Komponente somit tendenziell formveränderbar. Die Komponente kann dabei z.B. ein Band, ein Netz, ein Textil, eine Membran und/oder eine Kombinationen daraus sein. Die Verwendung einer elastischen Komponente ist vorteilhaft, da die Gesamtstabilität der Leit-Stütz-Struktur erhöht wird, die Komponente aufgrund ihrer elastischen Eigenschaft nicht oder nur unwesentlich einschnürend auf das Körperteil des Lebewesens wirkt und/oder mittels der elastischen Komponente ein Verrutschen der Leit-Stütz-Struktur weiter verhindert bzw. reduziert bzw. gehemmt wird.

Ferner ist vorstellbar, dass die Leit-Stütz-Struktur im Rahmen einer weiteren bevorzugten Ausführungsform als integraler Bestandteil eines Kleidungsstücks, insbesondere einer Hose, eines T-Shirts, eines Pullovers, einer Jacke, etc. ist.

Weiterhin ist denkbar, dass sich die Erfindung auch auf ein Verfahren zum Herstellen von mit einem Lebewesen koppelbaren Leit-Stütz-Strukturen bezieht, wobei eine Erfassung von Oberflächenverformungen von Körperflächen von Lebewesen zur Bestimmung geeigneter Anlageflächen für mit der Oberfläche der Lebewesen in Kontakt bringbare Leit-Stütz-Strukturen erfolgt bzw. dass sich die Erfindung auf eine Methode zur Herstellung einer Vorrichtung zur Befestigung unterschiedlicher Elemente am menschlichen Körper beziehen kann.

Das Herstellungsverfahren bzw. die Methode umfasst bevorzugt mindestens die Schritte des Ausrichtens zumindest eines Körperteils des Lebewesens gegenüber einer Erfassungseinrichtung, insbesondere einem 3D-Scanner, zum zumindest teilweisen Erfassen der Oberflächenverformungen von zumindest einem Oberflächenbereich des Körperteils, des Erfassens einer ersten Oberflächencharakteristik bzw. -form des Oberflächenbereichs mittels der Erfassungseinrichtung, wobei sich das Körperteil in einem ersten Belastungszustand, insbesondere einem Zustand entspannter Muskulatur, befindet, des Erfassens einer zweiten Oberflächencharakteristik bzw. -form des Oberflächenbereichs mittels der Erfassungseinrichtung, wobei sich das Körperteil in einem vom ersten Belastungszustand verschiedenen zweiten Belastungszustand, insbesondere einem Zustand angespannter Muskulatur, befindet, wobei bei der Erfassung in den verschiedenen Belastungszuständen denkbar ist, dass äußere Einflüsse berücksichtigt werden und/oder die Erfassung mit oder ohne einem Kontakt zu eventuellen Vorrichtungen durchgeführt wird, und des Bestimmens der sich zwischen dem ersten Belastungszustand und dem zweiten Belastungszustand ergebenden lokalen Oberflächenverformungen, wobei insbesondere ein 3D-Vergleich der Körperoberfläche der unterschiedlichen Scans vorgenommen wird.

In weiteren Schritten werden zusätzlich oder alternativ die für die optimale Anpassung einer Leit-Stütz-Struktur an ein Lebewesen geeigneten Flächen bzw. die Ortsverschiebungen bevorzugt extrahiert und besonders bevorzugt an spezifische Randbedingungen bzw. Aufgabenstellung, wie z.B. Belastungsszenarien, angepasst. Im Falle einer Orthese ist denkbar, dass Kräfte aufnehmbar bzw. aufzunehmen sind, um z.B. Valgus oder Innenrotationsbewegungen einzuschränken, zu verhindern oder zu beeinflussen, wofür die Kopplungsflächen bzw. der flächige Abschnitt des ersten Anteils gestalterisch und/oder fertigungstechnisch entsprechend anzupassen ist.

Bevorzugt erfolgt basierend auf den 3D-Daten bzw. CAD-Daten eine Fertigungssteuerung, wobei auch denkbar ist, dass die Daten als Vorlage für eine handwerkliche Herstellung dienen können. Die 3D-Scans der anwendungsspezifischen Körperregionen können nicht nur in unterschiedlichen Körperpositionen bzw. Körperstellungen bzw. Körperteilstellungen, insbesondere mit unterschiedlichen Belastungszuständen, sondern auch während einer Bewegung erfasst werden bzw. aus einer erfassten Bewegungsfolge extrahiert werden. Bevorzugt werden mindestens zwei oder genau zwei Scans durchgeführt, nämlich ein Anfangs- und Endscan der Bewegungsamplitude, wobei bevorzugt ein weiterer oder weitere, insbesondere genau zwei oder mehr als zwei, insbesondere genau drei oder mehr als drei, insbesondere genau vier oder mehr als vier, Scans zwischen der Anfangs- und Endposition getätigt werden.

Je nach Funktion der Leit-Stütz-Struktur wird gemäß einem zusätzlichen oder alternativen Verfahrensschritt bevorzugt ein Referenzsystem definiert bzw. festgelegt. Besonders bevorzugt erfolgt in einem weiteren Schritt ein 3D-Vergleich der Oberflächenverformungen oder indirekt oder direkt ein Volumenvergleich der Körperteile in den verschiedenen Belastungszuständen, wobei bevorzugt eine Definition der Ortsverschiebungen erfolgt. Dies kann unter Nutzung des Referenzsystems bzw. in Bezug dazu erfolgen.

Eine Fertigung einer solchen Leit-Stütz-Struktur erfolgt bevorzugt mittels eines generativen Verfahrens und/oder unter Einsatz einer additiven Fertigung, wobei im Herstellungsprozess insgesamt bevorzugt Laminierwerkzeuge und/oder Werkzeuge zum Nähen, Stricken, Weben, etc. eingesetzt werden. Es ist zudem denkbar, dass in einem zusätzlichen oder alternativen Verfahrensschritt, insbesondere in einem Fertigungsverfahrensschritt, ein Werkzeug erstellt wird, das bevorzugt in der späteren Leit-Stütz-Struktur verbleibt. Bevorzugt wird ein Herstellungsverfahren verwendet bzw. angewendet, bei dem die 3D-Daten direkt oder aufbereitet als Basis für die Fertigungssteuerung dienen. Weiterhin ist denkbar, dass Verbindungselemente als weitere Anbauteile zur Verbindung mit dem Körper des Lebewesens, insbesondere des menschlichen Körpers, vorgesehen sind. Diese Verbindungselemente dienen bevorzugt zur Ankopplung bzw. zum Verbinden mit bzw. zur Fixierung der Leit-Stütz-Struktur an dem Körperteil bzw. den Körperteilen und/oder zum Verbinden mit weiteren über die Leit-Stütz-Struktur am Lebewesen ankoppelbaren Elementen. Diese Verbindungselemente sind bevorzugt als Gurte, insbesondere als Klettgurte, flexible Klettgurte, Ledergurte, Textilgurte, Gewebegurte, oder als Schnürelemente ausgebildet. Die Verbindungselemente sind bewusst in rückseitigen Bereichen der Leit-Stütz-Struktur angeordnet bzw. anordenbar damit bevorzugt auf der Körperteilrückseite bzw. auf einer einem Verbindungsmittel gegenüberliegenden Seite eine Kraftaufbringung zur reibschlüssigen und/oder formschlüssigen Fixierung der Leit-Stütz-Struktur am Körper bzw. am Körperteil erfolgen kann.

Weitere Vorteile, Ziele und Eigenschaften vorliegender Erfindung werden anhand nachfolgender Beschreibung anliegender Zeichnungen erläutert, in welchen beispielhaft erfindungsgemäße Leit-Stütz-Strukturen dargestellt sind. Bauteile der erfindungsgemäßen Leit-Stütz-Strukturen, welche in den Figuren wenigstens im Wesentlichen hinsichtlich ihrer Funktion übereinstimmen, können hierbei mit gleichen Bezugszeichen gekennzeichnet sein, wobei diese Bauteile nicht in allen Figuren beziffert oder erläutert sein müssen.

Einzelne oder alle Darstellungen der im Nachfolgenden beschriebenen Figuren sind bevorzugt als Konstruktionszeichnungen anzusehen, d.h. die sich aus der bzw. den Figuren ergebenden Abmessungen, Proportionen, Funktionszusammenhänge und/oder Anordnungen entsprechen bevorzugt genau oder bevorzugt im Wesentlichen denen der erfindungsgemäßen Vorrichtung bzw. des erfindungsgemäßen Produkts.

Darin zeigen:
- Fig. 1: eine Darstellung von mittels des erfindungsgemäßen Verfahrens bestimmten Bereichen unterschiedlicher Oberflächenverformungen eines Körperteils einer Person;
- Fig. 2: eine schematische Darstellung einer Leit-Stütz-Struktur, die unter Berücksichtigung der in Fig. 1 gezeigten Bereiche unterschiedlicher Oberflächenverformung gestaltet ist;
- Fig. 3a: eine schematische laterale Darstellung des oberen Anteils der in Fig. 2 gezeigten Leit-Stütz-Struktur;
- Fig. 3b: eine schematische laterale Darstellung des unteren Anteils der in Fig. 2 gezeigten Leit-Stütz-Struktur;
- Fig. 4a: eine schematische frontal Darstellung des oberen Anteils der in Fig. 2 gezeigten Leit-Stütz-Struktur, wobei auch eine bevorzugt flexible und/oder elastische Komponente gezeigt ist;
- Fig. 4b: eine schematische frontal Darstellung des unteren Anteils der in Fig. 2 gezeigten Leit-Stütz-Struktur;
- Fig. 5a: eine schematische mediale Darstellung des oberen Anteils der in Fig. 2 gezeigten Leit-Stütz-Struktur;
- Fig. 5b: eine schematische mediale Darstellung des unteren Anteils der in Fig. 2 gezeigten Leit-Stütz-Struktur; und
- Fig. 6: ein Beispiel einer Vorrichtungsanordnung, mittels der das erfindungsgemäße Verfahren ausgeführt werden kann.

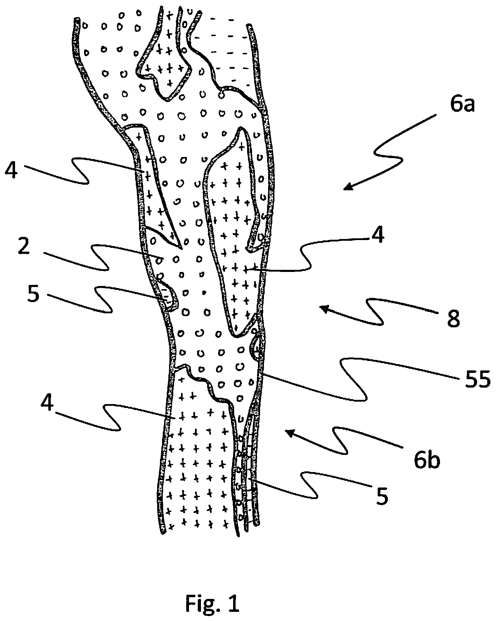

In Fig. 1 ist eine Darstellung eines Körperteils 6 (6a, 6b) gezeigt. Das Körperteil 6 ist ein menschliches Bein und weist bevorzugt zumindest teilweise einen Oberschenkel 6a und einen Unterschenkel 6b auf. Der Oberflächenbereich 8, der in dieser Darstellung fast den gesamten Oberschenkel 6a und mehr als die Hälfte des Unterschenkels 6b des Beins 6 umfasst, ist in verschiedene Zonen unterteilt. Im vorliegenden Beispiel sind genau drei voneinander verschiedene Zonenarten 2, 4, 5 gezeigt, wobei das Bezugszeichen 2 eine Zone geringer Volumenveränderungen bzw. einer sich gering verformenden Körperoberfläche und die Bezugszeichen 4, 5 Zonen hoher Volumenveränderungen bzw. sich stark verformender Körperoberflächen bezeichnet. Die Oberflächenverformungen in der dem ersten Oberflächenbereich zuordenbaren ersten Zone 2 sind bevorzugt geringer als die Oberflächenverformungen in der dem zweiten Oberflächenbereich zuordenbaren zweiten Zone 4 und bevorzugt geringer als die Oberflächenverformungen in der dem dritten Oberflächenbereich zuordenbaren dritten Zone 5, wobei die Oberflächenverformungen in der dritten Zone 5 in Bezug zu einem Zentrum des Körperteils entgegengerichtet zu den Oberflächenverformungen in der zweiten Zone 4 erfolgen. Die dritte Zone 5 kann daher eine Zone kennzeichnen, in der sich die Oberflächenverformung infolge einer Volumenabnahme ergibt und die zweite Zone 4 kann eine Zone kennzeichnen, in der sich die Oberflächenverformung infolge einer Volumenzunahme ergibt. Die erste Zone 2 ist bevorzugt eine Zone, in der keine bzw. im Wesentlichen keine Oberflächenverformung auftritt.

Das Bezugszeichen 55 kennzeichnet eine weitere Zone. Die weitere Zone 55 repräsentiert bevorzugt Bereiche, in denen zwar keine bzw. nur geringe Verformungen auftreten, die jedoch aufgrund der sehr nah unter der Oberfläche liegenden festen Bestandteile, wie z.B. Knochen oder Knorpel oder Implantate, als nicht zum Koppeln mit einer Leit-Stütz-Struktur 1 geeignet beurteilt werden. Daten zur Lage derartiger Zonen 55 sind bevorzugt vorgebbar und/oder aus einem Datenspeicher bzw. einer Datenbibliothek abrufbar und/oder vor der Oberflächenerfassung am Körper selbst zu kennzeichnen.

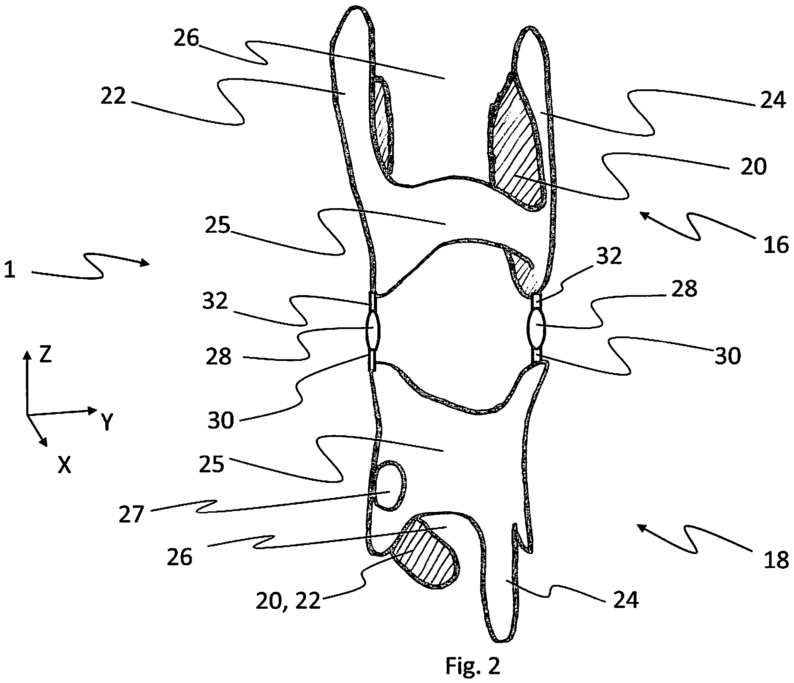

In Fig. 2 ist eine schematische perspektivische Darstellung einer bevorzugten Leit-Stütz-Struktur 1, insbesondere einer Bein- oder Knieorthese, gezeigt. Die Orthese 1 weist einen oberen Anteil 16 und einen unteren Anteil 18 auf. Der obere Anteil 16 ist bevorzugt derart ausgebildet, dass er an den Oberschenkel 6a des in Fig. 1 gezeigten Beins auf die erfindungsgemäße Weise ankoppelbar ist bzw. angekoppelt werden kann. Entsprechend den erfassten Oberflächenbereichen 2 und/oder 4 und/oder 5 ist bevorzugt ebenfalls der untere Anteil 18 der Orthese 1 ausgebildet, d.h. er ist derart gestaltet, dass er auf erfindungsgemäße Weise mit dem in Fig. 1 gezeigten Unterschenkel 6b koppelbar ist. Der obere Anteil 16 und der untere Anteil 18 sind bevorzugt über ein bzw. mehrere flexible Verbindungsstücke 28 und besonders bevorzugt über ein Gelenk 28 bzw. zwei Gelenke 28 miteinander verbunden. Das bzw. die Gelenke 28 können dabei direkt an dem oberen Anteil 16 und/oder an dem unteren Anteil 18 angeordnet bzw. ausgebildet sein, wobei sie bevorzugt mittels unteren Anbindungen 30 und/oder oberen Anbindungen 32 an dem ersten und zweiten Anteil 16, 18 angeordnet sind.

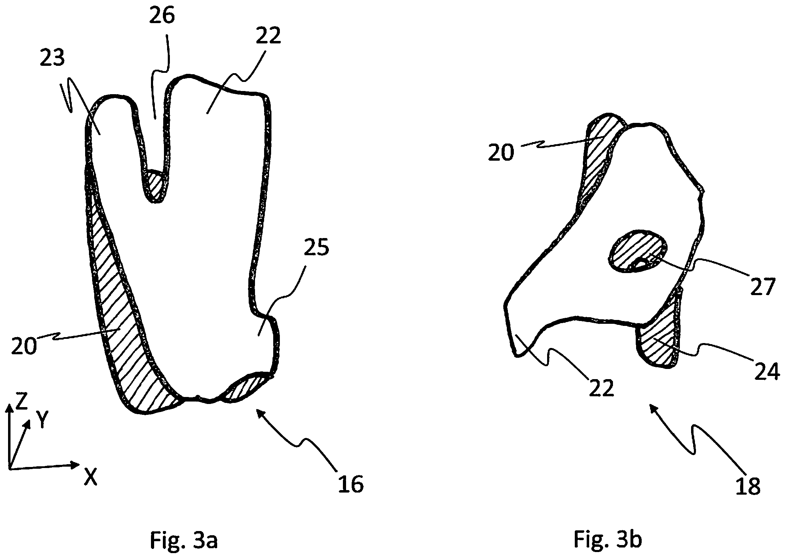

Der obere Anteil 16 weist bevorzugt mindestens einen ersten Teilbereich 22 und einen zweiten Teilbereich 24 zum seitlichen bzw. beidseitigen Umschließen des Oberschenkels 6a auf, wodurch der obere Anteil 16 bevorzugt formschlüssig an dem Oberschenkel 6a ankoppelbar ist. Die beiden Teilbereiche 22, 24 erstrecken sich bevorzugt in der Längsrichtung des Beins 6 bzw. des Oberschenkels 6a und sind bevorzugt durch ein Verbindungsmittel 25, das sich bevorzugt in Umfangsrichtung des Beines erstreckt, miteinander verbunden. Besonders bevorzugt bilden die Teilbereiche 22, 24 und eventuelle weitere Teilbereiche 23 (vgl. Fig. 3a) sowie das Verbindungsmittel 25 zusammen eine einstückig erzeugte Einheit. Es ist jedoch ebenfalls denkbar, dass einzelne dieser Elemente 22, 23, 24, 25 oder alle Elementen 22, 23, 24, 25 lösbar bzw. ersetzbar miteinander verbunden sind. Die Teilbereiche 22, 24 begrenzen gemäß dieser Darstellung eine Ausnehmung 26. Die Ausnehmung 26 stellt einen Bereich dar, in dem das Körperteil 6 bzw. der Oberschenkel 6a im Vergleich zu den durch die Teilbereiche 22, 24 überlagerten Bereichen größere Volumenveränderungen bzw. Oberflächenverformungen, insbesondere Ausdehnungen und Kontraktionen, erfahren kann, ohne dass eine Einschnürung des Körperteils 6 bei einer Volumenveränderung des Körperteils 6 bzw. bei einer Verformung der Körperteiloberfläche 4 resultiert. Auf der Innenseite ist zumindest ein Teilbereich 22, 23, 24 und bevorzugt der erste Teilbereich 22 und/oder der zweite Teilbereich 24 und besonders bevorzugt der erste Teilbereich 22, der zweite Teilbereich 24 und genau ein weiterer Teilbereich 23 oder mindestens ein weiterer Teilbereich 23 derart als flächiger Abschnitt 20 ausgebildet, dass er zumindest abschnittsweise flächig und zumindest mittelbar an der Oberfläche des Körperteils 6, insbesondere an Oberflächenbereichen 2, die zwischen zwei Belastungszuständen nur geringe bzw. keine bzw. im Wesentlichen keine Volumenveränderungen bzw. Oberflächenverformungen erfahren, anlegbar bzw. anordenbar ist. Es ist ebenfalls denkbar, dass sich das bevorzugt in Umfangsrichtung eines Körperteils 6 erstreckende Verbindungselement 25 auch zum Anliegen an der Körperoberfläche 2 bzw. zum in Kontakt bringen mit der Körperoberfläche 2 innenseitig flächig gestaltet ist. Sollte das Verbindungsmittel 25 im Bereich einer sich stark verformenden Körperoberfläche 4 angeordnet bzw. vorgesehen sein, so ist es bevorzugt derart gestaltet, dass es nicht bzw. nur unwesentlich mit der Körperoberfläche 4 in Kontakt bringbar ist.

In Bezug auf die Elemente 20, 22, 24, 25 des zweiten Abschnitts 18 sind die zuvor beschriebenen Ausprägungen bevorzugt analog anwendbar. Es ist der Darstellung ferner zu entnehmen, dass der erste Teilbereich 22 des zweiten Abschnitts 18 sich in eine andere Richtung erstreckt als der zweite Teilbereich 24 des zweiten Abschnitts 18. Die Längsachse des ersten Teilbereichs 22 erstreckt sich bevorzugt in Längsrichtung der Leit-Stütz-Struktur 1 und In Umfangsrichtung der Leit-Stütz-Struktur 1. Wohingegen der zweite Teilbereich 24 sich im Wesentlichen in der Längsrichtung Z der Leit-Stütz-Struktur 1 bzw. ausschließlich in der Längsrichtung Z der Leit-Stütz-Struktur 1 erstreckt. Es ist dennoch ebenfalls denkbar, dass sich in einer weiteren Ausführungsform die Längsachsen beider Teilbereiche 22, 24 zumindest teilweise in Umfangsrichtung erstrecken oder sich parallel bzw. im Wesentlichen parallel zur Längsachse Z der Leit-Stütz-Struktur 1 erstrecken.

Weiterhin ist der Darstellung entnehmbar, dass das Verbindungsmittel 25 des oberen Abschnitts 16 eine geringere Ausdehnung bzw. Erstreckung in der Längsrichtung der Leit-Stütz-Struktur 1 aufweist als das Verbindungsmittel 25 des zweiten Abschnitts 18. Es ist jedoch ebenfalls vorstellbar, dass dieses Verhältnis umgekehrt ist oder dass sich beide Verbindungsmittel 25 in der Längsrichtung der Leit-Stütz-Struktur 1 im Wesentlichen gleich weit oder genau gleich weit erstrecken. Die Teilbereiche 22 und 24 des zweiten Abschnitts 18 begrenzen bevorzugt ebenfalls eine Ausnehmung 26, die zum möglichst geringen Begrenzen bzw. Beschränken von Verformungen der Körperteiloberfläche 4 eines Lebewesens gestaltet ist. Es ist dieser Darstellung zu entnehmen, dass die Ausnehmung 26 des zweiten Abschnitts 18 gegenüber der Ausnehmung 26 des ersten Abschnitts 16 in Umfangsrichtung der Leit-Stütz-Struktur 1 versetzt bzw. rotiert ausgebildet ist. Je nach Einsatzgebiet, Lebewesen, etc. ist denkbar, dass die Ausnehmungen 26 des ersten und zweiten Abschnitts 16, 18 verdreht oder im Wesentlichen fluchtend bzw. genau fluchtend zueinander ausgerichtet sind.

Weiterhin lässt sich der Darstellung entnehmen, dass sowohl der obere als auch der untere Abschnitt 16, 18 bevorzugt je zwei bzw. genau zwei oder mindestens zwei Ausnehmungen 26 ausbilden. Die Anzahl der Ausnehmungen 16 ist bevorzugt von der Anzahl an Teilbereichen 22, 23, 24 abhängig, die bevorzugt je nach Einsatzbereich, Lebewesen und/oder Körperteil, etc. verschieden sein kann.

Das Bezugszeichen 27 kennzeichnet eine weitere Ausnehmung, insbesondere ein Loch. Die Form der Ausnehmung 27 kann dabei gerade Anteile und/oder sphärische Anteile aufweisen, wobei auch denkbar, ist dass sie nur gerade Anteile oder nur sphärische Anteile aufweist. Die Ausnehmung 27 kann zur Reduzierung des Gewichts, zum Abbau von Spannungen und/oder zum Freigeben von Oberflächenbereichen 4, insbesondere zum Ermöglichen beliebiger Oberflächenverformungen in dem freigegebenen bzw. nicht überlagerbaren Oberflächenbereich 4, ausgebildet sein.

In Fig. 3a ist eine Darstellung des oberen Abschnitts 16 in einer lateralen Ausrichtung gezeigt. Dabei ist ein weiterer Teilbereich 23 zu erkennen, der zusammen mit dem ersten Teilbereich 22 eine Ausnehmung 26 begrenzt. Weiterhin ist der Verbindungsbereich 25 derart ausgestaltet, dass er sich bevorzugt rechtwinklig von dem Zentrum bzw. der Längsrichtung der Leit-Stütz-Struktur 1 nach außen hin erstreckt. Diese Ausführungsform bietet den Vorteil, dass das Verbindungsmittel 25 das Körperteil 6 des Lebewesens derart beabstandet überlagert, dass es mit der Körperoberfläche 4 nicht in Kontakt bringbar ist. Bevorzugt sind die schraffiert dargestellten Bereiche flächige Abschnitte 20, die zum Anliegen an dem Lebewesen gestaltet sind.

In Fig. 3b ist eine Darstellung des unteren Abschnitts 16 in einer lateralen Ausrichtung gezeigt. Anhand dieser Darstellung lässt sich die Orientierung des ersten Teilbereichs 22 des unteren Abschnitts 18, insbesondere dessen teilweise Erstreckung in Längsrichtung und Umfangrichtung, erkennen. Weiterhin ist die bevorzugt runde bzw. ovale bzw. im Wesentlichen runde bzw. im Wesentlichen ovale Form der weiteren Ausnehmung 27 erkennbar. Es ist weiterhin denkbar, dass weitere Ausnehmungen 27, insbesondere Löcher, zusätzlich oder alternativ im zweiten Abschnitt 18 und/oder im ersten Abschnitt 16 ausgebildet sind bzw. ausgebildet werden.

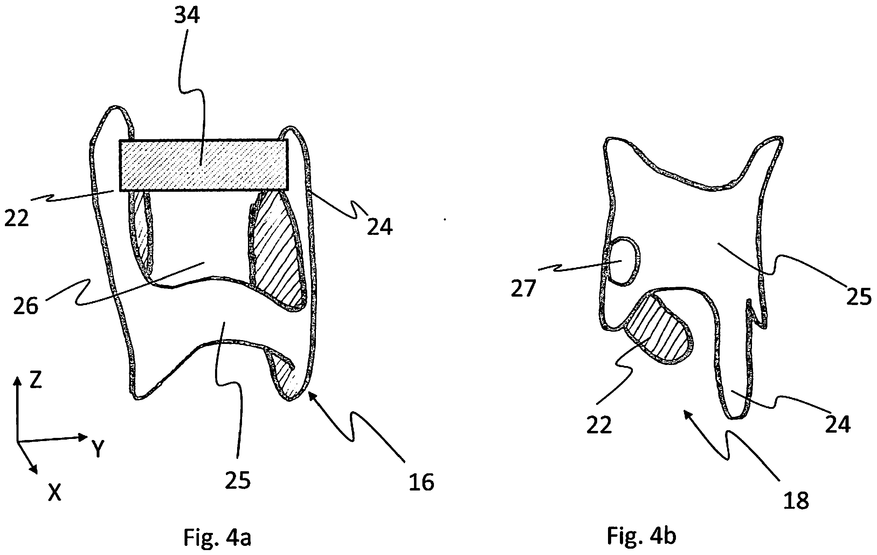

In Fig. 4a ist eine Darstellung des oberen Abschnitts 16 in einer frontalen Ausrichtung gezeigt. Der erste Teilbereich 22 und der zweite Teilbereich 24 des ersten Abschnitts 16 sind gemäß dieser Darstellung durch eine elastische Komponente 34, die z.B. als Gurt, Band, Gewebe, etc. ausgebildet sein kann, miteinander verbunden. Die elastische Komponente 34 kann dabei am oberen Teil bzw. im Bereich des oberen Endes der Leit-Stütz-Struktur 1 angeordnet oder ausgebildet sein, wobei auch denkbar ist, dass sie zusätzlich oder alternativ im mittleren Bereich der Ausnehmung 26 bzw. im unteren Bereich der Ausnehmung 26 angeordnet oder ausgebildet sein kann. Weiterhin ist vorstellbar, dass sich die elastische Komponente 34 im Wesentlichen über die gesamte Ausnehmung 26 erstreckt oder genau über die gesamte Ausnehmung 26 erstreckt. Bevorzugt ist die elastische Komponente 34 lösbar, insbesondere mittels Formschluss, Reibschluss oder Feldschluss, insbesondere mittels Klettverschluss, Reißverschluss, Druckknöpfen, etc., an der Leit-Stütz-Struktur 1, insbesondere an zwei Teilbereichen 22, 23, 24 angeordnet. Es ist jedoch ebenfalls denkbar, dass die elastische Komponente 34 fest, d.h. bevorzugt zerstörungsfrei unlösbar, mit der Leit-Stütz-Struktur 1 verbunden ist. Weiterhin ist zusätzlich oder alternativ vorstellbar, dass eine oder mehrere elastische Komponenten 34 auf der Rückseite der Leit-Stütz-Struktur 1 als Verbindungselemente zum Koppeln der Leit-Stütz-Struktur 1 mit dem Körperteil 6a bzw. dem Körper 6 angeordnet sind

In Fig. 4b ist eine weitere Darstellung des unteren Abschnitts 16 in einer frontalen Ausrichtung gezeigt.

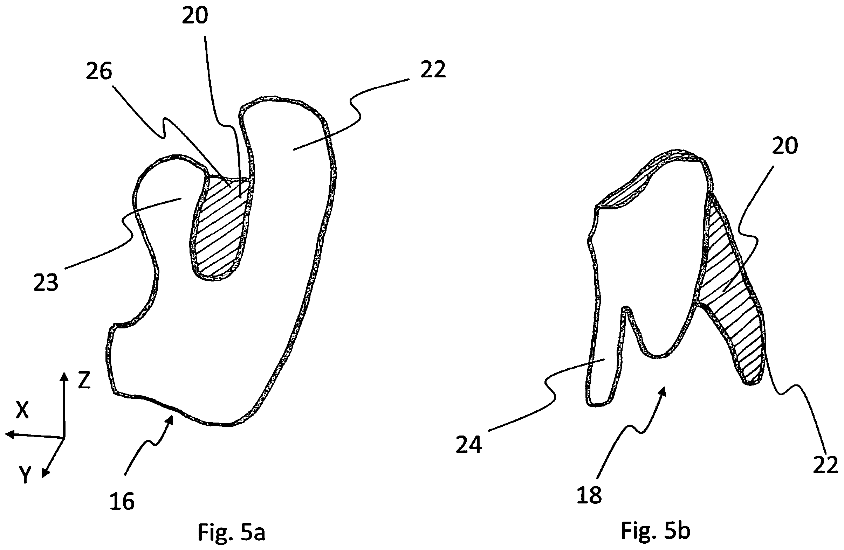

In Fig. 5a ist eine Darstellung des oberen Abschnitts 16 in einer medialen Ausrichtung gezeigt. Es ist dieser Darstellung zu entnehmen, dass z.B. der weitere Teilbereich 23 eine sphärische äußere Kontur aufweist. Diese Gestaltung resultiert aus einer bevorzugt möglichst genauen Analyse der in Fig. 1 gezeigten Oberflächenbereiche 2, 4, 5, damit eine bevorzugt möglichst große Fläche der sich gering verformenden Oberflächenbereiche 2 zur Ankopplung und somit zur Übertragung von Kräften verwendbar ist, da mit zunehmender Fläche größere Kräfte übertagbar sind bzw. bei konstanten Kräften ein noch größerer Komfort für das Lebewesen generierbar ist.

In Fig. 5b ist eine weitere Darstellung des unteren Abschnitts 16 in einer medialen Ausrichtung gezeigt. Die in Fig. 5b gezeigten Teilbereiche 22, 23, 24 sind bevorzugt unterschiedlich lang, unterschiedlich breit und bevorzugt jeweils mit einer unterschiedlichen äußeren Kontur gestaltet, wobei besonders bevorzugt denkbar ist, dass sich die Teilbereiche 22, 23, 24 in der Länge, der Kontur, der Erstreckungsrichtung und/oder der Breite abschnittsweise oder vollständig zumindest teilweise oder vollständig entsprechen. Die Teilbereiche 22, 23, 24 sind somit besonders bevorzugt in Abhängigkeit der jeweiligen Anwendung, des jeweiligen Lebewesens, etc. gestaltbar.

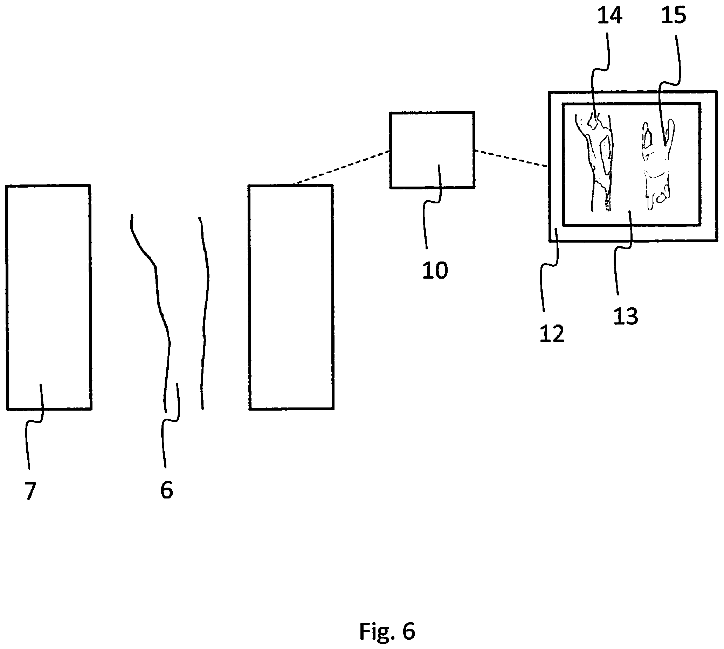

In Fig. 6 ist eine Anordnung gezeigt, mittels der bevorzugt das erfindungsgemäße Verfahren ausgeführt wird. Es ist hierbei jedoch denkbar, dass zusätzliche oder alternative Einrichtungen, Vorrichtungen, Mittel und/oder Methoden zum Einsatz kommen. Das Körperteil 6 befindet sich im Bereich einer Erfassungseinrichtung 7, die das Körperteil 6 zumindest zeitweise abschnittsweise und bevorzugt vollständig umgibt. Die von der Erfassungseinrichtung 7 erfassten Verformungswerte werden einer Prozessoreinrichtung 10 zugeführt, die bevorzugt zumindest signaltechnisch mit der Erfassungseinrichtung 7 verbunden ist. Die Prozessoreinrichtung 10 ermittelt bzw. berechnet die Verformungen, die Auftreten, wenn das Körperteil 6 des Lebewesens in verschiedenen Belastungszuständen untersucht und/oder gescannt und/oder erfasst bzw. vermessen wird.

Die von der Prozessoreinrichtung 10 generierten Ergebnisse sind bevorzugt über eine Anzeigeeinrichtung 12, die bevorzugt als Bildschirm 13 ausgebildet ist, ausgebbar. Auf dem Bildschirm 13 sind neben anderem zeitgleich oder nacheinander bevorzugt ein 3D-Model 14 des Körperteils 6, entsprechend den Ausführungen zu Fig. 1, und/oder ein anhand des 3D-Models 14 erzeugtes Model 15 einer erfindungsgemäßen Leit-Stütz-Struktur 1 zu entnehmen.

### Bezugszeichenliste

- 1: Leit-Stütz-Struktur
- 2: Sich gering verformende Körperoberfläche
- 4: Sich stark verformende Körperoberfläche
- 5: Stark verformende Körperoberfläche, insbesondere invers zur Körperoberfläche 4
- 6a: Erstes Körperteil
- 6b: Weiteres Körperteil
- 7: Erfassungseinrichtung
- 8: Oberflächenbereich
- 10: Prozessoreinrichtung
- 12: Anzeigeeinrichtung
- 13: Bildschirm
- 14: 3D-Model
- 15: Model der Leit-Stütz-Struktur
- 16: Erster Anteil
- 18: Zweiter Anteil
- 20: Flächiger Abschnitt
- 22: Erster Teilbereich
- 23: Weiterer Teilbereich
- 24: Zweiter Teilbereich
- 25: Verbindungselement
- 26: Ausnehmung
- 27: Weitere Ausnehmung
- 28: Gelenk
- 30: Unterseitige Anbindung
- 32: Oberseitige Anbindung
- 34: Elastische Komponente
- 55: Weitere Zone
- X: Tiefe
- Y: Breite
- Z: Länge

## Patentansprüche

1. Verfahren zum Erfassen von Oberflächenverformungen von Körperoberflächen von Lebewesen zur Bestimmung geeigneter Anlageflächen (2) für mit der Oberfläche der Lebewesen in Kontakt bringbare Leit-Stütz-Strukturen (1), wobei die Oberflächenverformungen durch Muskelvolumenveränderungen hervorgerufen werden,
das Verfahren mindestens umfassend die Schritte:
Ausrichten zumindest eines Körperteils (6a) des Lebewesens gegenüber einer Erfassungseinrichtung (7) zum zumindest teilweisen Erfassen der Oberflächenform von zumindest eines Oberflächenbereichs (8) des Körperteils (6a),
**gekennzeichnet durch** die Schritte
Erfassen einer ersten Oberflächenform des Oberflächenbereichs (8) mittels der Erfassungseinrichtung (7), wobei sich das Körperteil (6a) in einem ersten Belastungszustand, insbesondere einem Zustand entspannter Muskulatur, befindet,
Erfassen einer zweiten Oberflächenform des Oberflächenbereichs (8) mittels der Erfassungseinrichtung (7), wobei sich das Körperteil (6a) in einem vom ersten Belastungszustand verschiedenen zweiten Belastungszustand, insbesondere einem Zustand angespannter Muskulatur, befindet,
Bestimmen der sich zwischen dem ersten Belastungszustand und dem zweiten Belastungszustand ergebenden lokalen Oberflächenverformungen (2, 4, 5).

2. Verfahren nach Anspruch 1,
**dadurch gekennzeichnet, dass**
beim Bestimmen der lokalen Oberflächenverformungen ein Abgleich der erfassten lokalen Oberflächenverformungen mit zumindest einem definierten Schwellenwert oder einer definierten Schwellenwertfunktion durchgeführt wird, wobei daraus resultierende Verformungswerte einerseits des definierten Schwellenwertes oder der definierten Schwellenwertfunktion, insbesondere unterhalb des definierten Schwellenwertes oder der definierten Schwellenwertfunktion, als Werte geringer Oberflächenverformung, insbesondere geringer Expansion oder Kontraktion, beschreibbar sind und andererseits des definierten Schwellenwertes oder der definierten Schwellenwertfunktion, insbesondere oberhalb des definierten Schwellenwerts oder der definierten Schwellenwertfunktion, als Werte hoher Oberflächenverformung beschreibbar sind.

3. Verfahren nach einem der Ansprüche 1 oder 2,
die Bestimmung der lokalen Oberflächenverformungen und/oder der Abgleich der erfassten Oberflächenverformungen durch eine Prozessoreinrichtung (10) bewirkt wird.

4. Verfahren nach Anspruch 2 oder 3
**dadurch gekennzeichnet, dass**
eine zu den Verformungswerten und/oder zu den einerseits des Schwellenwertes oder der Schwellenwertfunktion zuordenbaren Ortsbereichen korrespondierende Darstellung zum Anzeigen unterschiedlicher Verformungsgrade an unterschiedlichen Positionen des Oberflächenbereichs (8) erzeugt und/oder visualisiert wird.

5. Verfahren nach Anspruch 2 bis 4
**dadurch gekennzeichnet, dass**
die Verformungswerte und/oder die einerseits des Schwellenwertes oder der Schwellenwertfunktion zuordenbaren Ortsbereiche in Daten zur Erstellung eines 3D-Modells überführt werden.

6. Computer implementiertes Verfahren nach einem der Ansprüche 1 bis 5.

7. Leit-Stütz-Struktur (1), insbesondere Orthese, zum Koppeln mit einem Lebewesen zum Übertragen von in einen ersten Anteil (16) der Leit-Stütz-Struktur (1) einleitbaren Kräften auf einen zweiten vom ersten Anteil (16) beabstandeten Anteil (18) der Leit-Stütz-Struktur (1),
wobei der erste Anteil (16) zum zumindest mittelbaren und zumindest abschnittsweise flächigen Anliegen (20) an einem Oberflächenbereich (8) eines Körperteils (6a) eines Lebewesens ausgebildet ist,
**dadurch gekennzeichnet, dass**
zumindest der flächige Abschnitt (20) des ersten Anteils (16) zum Anliegen an dem Körperteil (6a) derart ausgebildet ist, dass
er in einer für die Funktionserfüllung der Leit-Stütz-Struktur (1) vorgesehenen definierten Ausrichtung gegenüber dem Körperteil (6a) an Oberflächenbereiche (2) anlegbar ist, die beim Übergang von einem ersten Belastungszustand in einen zweiten Belastungszustand Oberflächenverformungen aufgrund Muskelvolumenänderungen aufweisen, die während desselben Übergangs geringer sind als Oberflächenverformungen weiterer Oberflächenbereiche (4, 5) des Körperteils (6a)
oder
er in einer für die Funktionserfüllung der Leit-Stütz-Struktur (1) vorgesehenen definierten Ausrichtung gegenüber dem Körperteil (6a) an Oberflächenbereiche (4, 5) anlegbar ist, die beim Übergang von einem ersten Belastungszustand in einen zweiten Belastungszustand Oberflächenverformungen aufgrund Muskelvolumenänderungen aufweisen, die während desselben Übergangs höher sind als Oberflächenverformungen weiterer Oberflächenbereiche (2) des Körperteils (6a).

8. Leit-Stütz-Struktur nach Anspruch 7,
**dadurch gekennzeichnet, dass**
in Abhängigkeit von mindestens zwei definierten Schwellenwerten oder Schwellenwertfunktionen mindestens drei Oberflächenbereiche (2, 4, 5) Zonen unterschiedlicher Oberflächenverformungen zuordenbar sind,
insbesondere sind die Oberflächenverformungen in der dem ersten Oberflächenbereich (2) zuordenbaren ersten Zone geringer als die Oberflächenverformungen in der dem zweiten Oberflächenbereich (4) zuordenbaren zweiten Zone und geringer als die Oberflächenverformungen in der dem dritten Oberflächenbereich (5) zuordenbaren dritten Zone, wobei die Oberflächenverformungen in der dritten Zone in Bezug zu einem Zentrum des Körperteils entgegengerichtet zu den Oberflächenverformungen in der zweiten Zone erfolgen.

9. Leit-Stütz-Struktur nach Anspruch 7 oder 8,
**dadurch gekennzeichnet, dass**
der flächige Abschnitt (20) derart gestaltet ist, dass er überwiegend mit Oberflächenbereichen (2) des Körperteils (6a) in Kontakt bringbar ist, die sich beim Übergang von einem ersten Belastungszustand in einen zweiten Belastungszustand geringer verformen als die weiteren Oberflächenbereiche (4, 5) des Körperteils (6a).

10. Leit-Stütz-Struktur nach Anspruch 7 oder 8,
**dadurch gekennzeichnet, dass**
der flächige Abschnitt (20) derart gestaltet ist, dass er mit Oberflächenbereichen (2) des Körperteils (6a) in Kontakt bringbar ist, die sich beim Übergang von einem ersten Belastungszustand in einen zweiten Belastungszustand geringer verformen als die weiteren Oberflächenbereiche (4, 5) des Körperteils (6a).

11. Leit-Stütz-Struktur nach einem der Ansprüche 7 bis 10,
**dadurch gekennzeichnet, dass**
der zweite Anteil (18) der Leit-Stütz-Struktur (1) derart an einem weiteren Körperteil (6b), insbesondere an einem Unterschenkel, anlegbar ist, dass er in der für die Funktionserfüllung der Leit-Stütz-Struktur (1) vorgesehenen definierten Ausrichtung gegenüber dem Körperteil (6a), insbesondere einem Oberschenkel, an Oberflächenbereiche (2) des weiteren Körperteils (6b) anlegbar ist, die beim Übergang von einem ersten Belastungszustand in einen zweiten Belastungszustand Oberflächenverformungen aufweisen, die gegenüber Oberflächenverformungen weiterer Oberflächenbereiche (4,5) des weiteren Körperteils (6b) während desselben Übergangs geringer sind,
oder
an Oberflächenbereiche (4, 5) des weiteren Körperteils (6b) anlegbar ist, die beim Übergang von einem ersten Belastungszustand in einen zweiten Belastungszustand Oberflächenverformungen aufweisen, die gegenüber Oberflächenverformungen weiterer Oberflächenbereiche (2) des weiteren Körperteils (6b) während desselben Übergangs höher sind.

12. Leit-Stütz-Struktur nach einem der Ansprüche 7 bis 11,
**dadurch gekennzeichnet, dass**
die Form des flächigen Abschnitts (20) in Abhängigkeit von einem Verfahren nach einem der Ansprüche 1 bis 6 bestimmbar ist.

13. Leit-Stütz-Struktur nach einem der Ansprüche 7 bis 11,
**dadurch gekennzeichnet, dass**
die Form des flächigen Abschnitts (20) in Abhängigkeit von Personengruppen und/oder Körperteilgruppen zuordenbaren und in einer Datensammlung vorgehaltenen lokalen Oberflächenverformungsdaten, die nach einem der Ansprüche 1 bis 6 bestimmt sind, bestimmbar ist.

14. Leit-Stütz-Struktur nach einem der Ansprüche 7 bis 13
**dadurch gekennzeichnet, dass**
der flächige Abschnitt (20) zwei Teilbereiche (22, 24) ausbildet, von denen der Oberflächenbereich (8) des Körperteils (6a) zumindest teilweise umschließbar ist und zwischen denen eine Ausnehmung (26) ausgebildet ist, wobei ein der Ausnehmung (26) zuordenbarer Oberflächenbereich (4, 5) beim Übergang von einem ersten Belastungszustand in einen zweiten Belastungszustand größere Oberflächenverformungen aufweist als die von dem flächigen Abschnitt (20) überlagerbaren Oberflächenbereiche (2)
oder
ein der Ausnehmung (26) zuordenbarer Oberflächenbereich (4, 5) beim Übergang von einem ersten Belastungszustand in einen zweiten Belastungszustand geringere Oberflächenverformungen aufweist als die von dem flächigen Abschnitt (20) überlagerbaren Oberflächenbereiche (2).

15. Leit-Stütz-Struktur nach Anspruch 14
**dadurch gekennzeichnet, dass**
die zwei Teilbereiche (22, 24) des flächigen Abschnitts (20) mittels einer sich im Bereich der Ausnehmung (26) erstreckenden Komponente (26), insbesondere einer flexible Komponente, verbunden sind.

## Claims

1. A method for detecting surface deformations of body surfaces of living beings for determining suitable contact surfaces (2) for guiding and supporting structures (1) which can be brought into contact with the surface of the living beings, the surface deformations being brought about by changes in muscle volume, the method comprising at least the steps:
orienting at least one body part (6a) of the living being with respect to a detection device (7) for the at least partial detection of the surface shape of at least one surface area (8) of the body part (6a),
**characterised by** the steps
detecting a first surface shape of the surface area (8) by means of the detection device (7), wherein the body part (6a) is in a first load state, in particular a state with the muscles relaxed,
detecting a second surface shape of the surface area (8) by means of the detection device (7), wherein the body part (6a) is in a second load state different from the first load state, in particular a state with the muscles tensed,
determining the local surface deformations (2, 4, 5) that result between the first load state and the second load state.

2. The method according to claim 1,
**characterised in that**
when the local surface deformations are determined, a comparison of the detected local surface deformations with at least one defined threshold value or one defined threshold value function is carried out, wherein resulting deformation values to one side of the defined threshold value or of the defined threshold value function, in particular below the defined threshold value or the defined threshold value function, can be described as values of low surface deformation, in particular lower expansion or contraction, and resulting deformation values to the other side of the defined threshold value or of the defined threshold value function, in particular above the defined threshold value or the defined threshold value function, can be described as values of high surface deformation.

3. The method according to one of claims 1 or 2,
**characterised in that**
the determination of the local surface deformations and/or the comparison of the detected surface deformations is effected by a processor device (10).

4. The method according to claim 2 or 3,
**characterised in that**
a representation corresponding to local areas which can be assigned to the deformation values and/or to one side of the threshold value or threshold value function is generated and/or visualised to show different degrees of deformation at different positions of the surface area (8).

5. The method according to claims 2 to 4,
**characterised in that**
the deformation values and/or the local areas which can be assigned to one side of the threshold value or threshold value function are converted into data to create a 3D model.

6. A computer-implemented method according to any one of claims 1 to 5.

7. A guiding and supporting structure (1), in particular orthosis, for coupling to a living being to transmit forces which can be introduced into a first portion (16) of the guiding and supporting structure (1) to a second portion (18), at a distance from the first portion (16), of the guiding and supporting structure (1), the first portion (16) being designed to bear (20) flat, at least indirectly and at least in some sections, against a surface area (8) of a body part (6a) of a living being,
**characterised in that**
at least the flat section (20) of the first portion (16) is designed to bear against the body part (6a) in such a manner that
said flat section can be placed in a defined orientation, intended to provide the function of the guiding and supporting structure (1), relative to the body part (6a) against surface areas (2) which exhibit surface deformations, owing to changes in muscle volume on transitioning from a first load state to a second load state, which are smaller during the same transition than surface deformations of other surface areas (4, 5) of the body part (6a), or said flat section can be placed in a defined orientation, intended to provide the function of the guiding and supporting structure (1), relative to the body part (6a) against surface areas (4, 5) which exhibit surface deformations, owing to changes in muscle volume on transitioning from a first load state to a second load state, which are greater during the same transition than surface deformations of other surface areas (2) of the body part (6a).

8. The guiding and supporting structure according to claim 7,
**characterised in that**
depending on at least two defined threshold values or threshold value functions, at least three surface areas (2, 4, 5) can be assigned to zones of different surface deformations,
in particular, the surface deformations in the first zone which can be assigned to the first surface area (2) are smaller than the surface deformations in the second zone which can be assigned to the second surface area (4) and smaller than the surface deformations in the third zone which can be assigned to the third surface area (5), wherein the surface deformations in the third zone take place in the opposite direction to the surface deformations in the second zone in relation to a centre of the body part.

9. The guiding and supporting structure according to claim 7 or 8,
**characterised in that**
the flat section (20) is designed such that it can be brought into contact predominantly with surface areas (2) of the body part (6a) which deform less during transition from a first load state to a second load state than the other surface areas (4, 5) of the body part (6a).

10. The guiding and supporting structure according to claim 7 or 8,
**characterised in that**
the flat section (20) is designed such that it can be brought into contact with surface areas (2) of the body part (6a) which deform less during transition from a first load state to a second load state than the other surface areas (4, 5) of the body part (6a).

11. The guiding and supporting structure according to any one of claims 7 to 10,
**characterised in that**
the second portion (18) of the guiding and supporting structure (1) can be placed against another body part (6b), in particular against a lower leg, in such a manner that said second portion can be placed in the defined orientation, intended to provide the function of the guiding and supporting structure (1), relative to the body part (6a), in particular an upper leg, against surface areas (2) of the other body part (6b) which exhibit surface deformations on transitioning from a first load state to a second load state which are smaller during the same transition than surface deformations of other surface areas (4, 5) of the other body part (6b),
or
said second portion can be placed against surface areas (4, 5) of the other body part (6b) which exhibit surface deformations on transitioning from a first load state to a second load state which are greater during the same transition than surface deformations of other surface areas (2) of the other body part (6b) .

12. The guiding and supporting structure according to any one of claims 7 to 11,
**characterised in that**
the shape of the flat section (20) can be determined on the basis of a method according to any one of claims 1 to 6.

13. The guiding and supporting structure according to any one of claims 7 to 11,
**characterised in that**
the shape of the flat section (20) can be determined on the basis of local surface deformation data which can be assigned to groups of persons and/or to groups of body parts, is stored in a data collection, and is determined according to any one of claims 1 to 6.

14. The guiding and supporting structure according to any one of claims 7 to 13,
**characterised in that**
the flat section (20) forms two part-areas (22, 24) by which the surface area (8) of the body part (6a) can be at least partially enclosed and between which a recess (26) is formed, wherein a surface area (4, 5) which can be assigned to the recess (26) exhibits greater surface deformations on transitioning from a first load state to a second load state than the surface areas (2) which can be covered by the flat section (20),
or
a surface area (4, 5) which can be assigned to the recess (26) exhibits smaller surface deformations on transitioning from a first load state to a second load state than the surface areas (2) which can be covered by the flat section (20).

15. The guiding and supporting structure according to claim 14,
**characterised in that**
the two part-areas (22, 24) of the flat section (20) are connected by means of a component (26), in particular a flexible component, which extends in the region of the recess (26).

## Revendications

1. Procédé permettant de saisir des déformations de surface de surfaces corporelles d'un être vivant pour déterminer des surfaces d'appui appropriées (2) pour des structures de guidage et de support (1) pouvant être mises en contact avec la surface de l'être vivant, les déformations de surface étant causées par des modifications de volume musculaire, le procédé comprenant au moins les étapes suivantes :
orientation au moins d'une partie du corps (6a) de l'être vivant par rapport à un dispositif de saisie (7) pour saisir au moins partiellement la forme de surface d'au moins une zone de surface (8) de la partie du corps (6a),
**caractérisé par** les étapes
de saisie d'une première forme de surface de la zone de surface (8) au moyen du dispositif de saisie (7), la partie du corps (6a) se trouvant dans un état de contrainte, notamment un état de musculature détendue,
de saisie d'une deuxième forme de surface de la zone de surface (8) au moyen du dispositif de saisie (7), la partie du corps (6a) se trouvant dans un deuxième état de contrainte différent du premier état de contrainte, notamment un état de musculature tendue,
de détermination des déformations de surface (2, 4, 5) locales résultantes entre le premier état de contrainte et le deuxième état de contrainte.

2. Procédé selon la revendication 1,
**caractérisé en ce que**
lors de la détermination des déformations de surfaces locales une harmonisation des déformations de surfaces locales saisies est effectuée avec au moins une valeur seuil définie ou une fonction de valeur seuil définie, les valeurs de déformations en résultant d'une part de la valeur seuil définie ou de la fonction de valeur seuil définie, notamment en dessous de la valeur seuil définie ou de la fonction de valeur seuil définie pouvant être décrites en tant que valeurs de déformation de surface minime, notamment d'expansion ou de contraction minime et d'autre part de la valeur seuil définie ou de la fonction de valeur seuil définie, notamment au-dessus de la valeur seuil définie ou de la fonction de valeur seuil définie pouvant être décrites en tant que valeurs de déformation de surface plus importante.

3. Procédé selon l'une quelconque des revendications 1 ou 2,
**caractérisé en ce que**
la détermination des déformations de surface locales et/ou l'harmonisation des déformations de surfaces saisies est réalisée par un dispositif à processeur (10) .

4. Procédé selon la revendication 2 ou 3,
**caractérisé en ce**
**qu'**une représentation correspondant aux valeurs de déformation et/ou aux zones locales attribuables d'une part de la valeur seuil ou de la fonction de valeur seuil est produite et/ou visualisée pour afficher des degrés de déformation différents à des positions différentes de la zone de surface (8).

5. Procédé selon les revendications 2 à 4,
**caractérisé en ce que**
les valeurs de déformation et/ou les zones locales attribuables d'une part de la valeur seuil ou de la fonction de valeur seuil sont transformées en données pour établir un modèle tridimensionnel.

6. Procédé mis en oeuvre de façon informatisée selon l'une quelconque des revendications 1 à 5.

7. Structure de guidage et de support (1), notamment orthèse, à appliquer à un être vivant pour transmettre des forces pouvant être introduites dans une première partie (16) de la structure de guidage et de support (1) à une deuxième partie (18) de la structure de guidage et de support (1) distante de la première partie (16), la première partie (16) étant constituée pour l'application en surface (20) au moins indirecte et au moins par section à une zone de surface (8) d'une partie de corps (6a) d'un être vivant,
**caractérisée en ce**
**qu'**au moins la section en surface (20) de la première partie (16) est constituée pour application à la partie du corps (6a) de telle manière
**qu'**elle peut être appliquée à des zones de surfaces (2) dans une orientation définie prévue pour remplir la fonction de la structure de guidage et de support (1) par rapport à la partie du corps (6a), qui comportent lors du passage d'un premier état de contrainte à un deuxième état de contrainte des déformations de surface en raison de modifications de volume musculaire, qui sont plus faibles pendant ledit passage que les déformations de surface d'autres zones de surface (4, 5) de la partie du corps (6a) ou qu'elle peut être appliquée à des zones de surface (4, 5) dans une orientation définie prévue pour remplir la fonction de la structure de guidage et de support (1) par rapport à la partie du corps (6a) qui comportent lors du passage d'un premier état de contrainte à un deuxième état de contrainte des déformations de surface en raison de modifications de volume musculaire qui sont plus importantes pendant ledit passage que les déformations de surface d'autres zones de surface (2) de la partie du corps (6a).

8. Structure de guidage et de support selon la revendication 7,
**caractérisée en ce**
**qu'**en fonction d'au moins deux valeurs seuils ou fonctions de valeur seuil définies des zones de déformations de surface différentes peuvent être attribuées à au moins trois zones de surface (2, 4, 5),
notamment les déformations de surface dans la première zone attribuable à la première zone de surface (2) sont plus faibles que les déformations de surface dans la deuxième zone attribuable à la deuxième zone de surface (4) et plus faibles que les déformations de surface dans la troisième zone attribuable à la troisième zone de surface (5), les déformations de surface ayant lieu dans la troisième zone en référence à un centre de la partie du corps opposée aux déformations de surface dans la deuxième zone.

9. Structure de guidage et de support selon la revendication 7 ou 8,
**caractérisée en ce que**
la section en surface (20) est structurée de telle manière qu'elle peut être surtout mise en contact avec les zones de surface (2) de la partie du corps (6a) qui se déforment plus faiblement lors du passage d'un premier état de contrainte à un deuxième état de contrainte que les autres zones de surface (4, 5) de la partie du corps (6a).

10. Structure de guidage et de support selon la revendication 7 ou 8,
**caractérisée en ce que**
la section en surface (20) est structurée de telle manière qu'elle peut être mise en contact avec les zones de surface (2) de la partie du corps (6a) qui se déforment lors du passage d'un premier état de contrainte à un deuxième état de contrainte plus faiblement que les autres zones de surface (4, 5) de la partie du corps (6a).

11. Structure de guidage et de support selon l'une quelconque des revendications 7 à 10,
**caractérisée en ce que**
la deuxième partie (18) de la structure de guidage et de support (1) peut être appliquée à une autre partie du corps (6b), notamment à une jambe, de telle manière qu'elle peut être appliquée dans l'orientation définie prévue pour remplir la fonction de la structure de guidage et de support (1) par rapport à la partie du corps (6a), notamment à une jambe, à des zones de surface (2) de l'autre partie du corps (6b), qui comportent lors du passage d'un premier état de contrainte à un deuxième état de contrainte des déformations de surface qui sont plus faibles par rapport aux déformations de surface d'autres zones de surface (4, 5) de l'autre partie du corps (6b), pendant ledit passage,
ou
peut être appliquée à des zones de surface (4, 5) de l'autre partie du corps (6b), qui comportent lors du passage d'un premier état de contrainte à un deuxième état de contrainte des déformations de surface qui sont plus importantes pendant ledit passage par rapport à des déformations de surface d'autres zones de surface (2) de l'autre partie du corps (6b).

12. Structure de guidage et de support, selon l'une quelconque des revendications 7 à 11,
**caractérisée en ce que**
la forme de la section en surface (20) peut être déterminée en fonction d'un procédé selon l'une quelconque des revendications 1 à 6.

13. Structure de guidage et de support, selon l'une quelconque des revendications 7 à 11,
**caractérisée en ce que**
la forme de la section en surface (20) peut être déterminée en fonction des données de déformation de surface locales attribuables de groupes de personnes et/ou de groupes de parties du corps et détenues dans une collecte de données, qui peuvent être déterminées selon l'une quelconque des revendications 1 à 6.

14. Structure de guidage et de support, selon l'une quelconque des revendications 7 à 13,
**caractérisée en ce que**
la section en surface (20) est constituée de deux parties de zone (22, 24) dont la zone de surface (8) de la partie du corps (6a) peut être entourée au moins en partie et entre lesquelles est constitué un évidement (26) et une zone de surface (4, 5) attribuable à l'évidement (26) comportant lors du passage d'un premier état de contrainte à un deuxième état de contrainte des déformations de surface plus importantes que les zones de surface (2) superposables par la section en surface (20),
ou
une zone de surface (4, 5) attribuable à l'évidement (26) comportant lors du passage d'un premier état de contrainte à un deuxième état de contrainte des déformations de surface plus faibles que les zones de surface (2) superposables par la section en surface (20) .

15. Structure de guidage et de support, selon la revendication 14,
**caractérisée en ce que**
les deux parties de zone (22, 24) de la section en surface (20) sont reliées au moyen d'un composant (26) s'étendant dans la zone de l'évidement (26), notamment d'un composant flexible.
